(19) Europäisches Patentamt / European Patent Office / Office européen des brevets

(11) **EP 3 022 613 B1**

(12) **EUROPEAN PATENT SPECIFICATION**

(45) Date of publication and mention
of the grant of the patent:
**29.12.2021 Bulletin 2021/52**

(51) Int Cl.:
*A61B 5/02* *(2006.01)*          *A61B 5/1171* *(2016.01)*
*A61B 5/00* *(2006.01)*          *G06K 9/00* *(2006.01)*

(21) Application number: **14811031.5**

(22) Date of filing: **05.06.2014**

(86) International application number:
**PCT/US2014/041004**

(87) International publication number:
**WO 2014/200788 (18.12.2014 Gazette 2014/51)**

(54) **BIOMETRIC IDENTIFICATION METHOD USING PULSE WAVEFORM**

BIOMETRISCHES IDENTIFIKATIONSVERFAHREN MIT PULSWELLENFORM

MÉTHODE D'IDENTIFICATION BIOMÉTRIQUE UTILISANT UNE FORME D'ONDE D'IMPULSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.06.2013 US 201313916818**

(43) Date of publication of application:
**25.05.2016 Bulletin 2016/21**

(73) Proprietor: **Lifeloc Technologies, Inc.
Wheat Ridge, Colorado 80033 (US)**

(72) Inventors:
• **PHILLIPS, Brian, Kirby
Lakewood, CO 80228 (US)**
• **WILSON, Geoffrey, A.
Roseburg, OR 97471 (US)**

(74) Representative: **Goddard, Frances Anna et al
Mathisen & Macara LLP
Communications House
South Street
Staines-upon-Thames TW18 4PR (GB)**

(56) References cited:
WO-A2-2012/138663     WO-A2-2012/138663
US-A1- 2003 135 097     US-A1- 2006 013 445
US-A1- 2012 253 154

• **MEI CHEN ET AL: "Biometrics with physical exercise using Laser Doppler Vibrometry measurements of the carotid pulse", BIOMETRICS, IDENTITY AND SECURITY (BIDS), 2009 INTERNATIONAL CONFERENCE ON, IEEE, PISCATAWAY, NJ, USA, 22 September 2009 (2009-09-22), pages 1-6, XP031707243, ISBN: 978-1-4244-5276-7**
• **None**

**Description**

**BACKGROUND OF THE INVENTION**

**Field of the Invention.** The present invention relates generally to the field of biometric identity confirmation. More specifically, the present invention discloses a system for biometric identity confirmation based on analysis of pulse waveform data for a test subject.

**Background of the Invention.** Biometric identification is the process of recognizing or rejecting an unknown person as a particular member of a previously characterized set, based on biological measurements. The ideal biometric characterization is specific to the individual, difficult to counterfeit, robust to metabolic fluctuations, insensitive to external conditions, easily measured, and quickly processed.

Fingerprint, retinal, iris, and facial scans are well-known biometric identification techniques relying on image processing. Images are two-dimensional, requiring sophisticated and computationally intensive algorithms, the analysis of which is often complicated by random orientation and variable scaling. Voice recognition is an example of biometric identification amenable to time series analysis, an inherently simpler one-dimensional process.

The simplest biometric identifiers can be expressed as a single parameter, such as height or weight. Single parameter identifiers have been the only quantitative means of identification throughout most of history. The price of simplicity is the loss of specificity, and in the case of weight, the lack of constancy over time. Nevertheless, single-parameter biometrics remain effective identifying factors, as is obvious from their continued use.

Identity tracking/confirmation is the process of following the whereabouts of a known subject moving unpredictably among similar individuals, perhaps with deceptive intent. Tracking/confirmation is somewhat simpler than identification, because it merely requires distinguishing the subject from all others rather than distinguishing every individual from every other, and because continuous rather than episodic data are available. Biometric identity tracking/confirmation is the continuous verification that a body-mounted sensor has remained on the subject, and has not been surreptitiously transferred to an impostor. For the purposes of this application, the term "biometric identification" should be broadly construed to encompass both biometric identification in its narrower sense, as described above, and identity tracking/confirmation.

US2003/135097 discloses a method for verifying identity based on signals such as heart rate variability and/or respiration processes. For example, the method may include averaging normalized waves extracted from a short segment of pulse oximeter data, in which the signal may be processed using noise filtering, signal decomposition, and / or feature extraction. Information about the shape of the pulse wave can be captured in the time domain through autoregression modeling.

WO 2012/138663 A2 relates to a method for biometric identity confirmation of a subject having a pulse and a respiratory cycle wherein a second derivative of the pulse wave data with respect to time is used to identify pulse cycle start points for synchronous averaging of multiple pulse cycles when analysing pulse wave data to confirm whether the identity of a test subject matches a known subject.

**SUMMARY OF THE INVENTION**

This invention provides a method for biometric identity confirmation of a subject having a pulse as claims in the attached claims. The method is based on pulse waveform data for the test subject. During an initial enrollment mode, pulse waveform data for a known subject are used to generate subject characterization data for the known subject. The subject characterization data includes an exemplar created by synchronous averaging of pulse waveform data over multiple pulse cycles. A number of trigger candidate are identified for the start point of each pulse cycle. The time delay between trigger candidates is analyzed to discard false trigger candidate and identify true candidates, which are then used as the start points for each pulse cycle in synchronous averaging of the pulse waveform data. During a subsequent identity authentication mode, pulse waveform data for a test subject are analyzed using the subject characterization data to confirm whether the identity of the test subject matches the known subject.

These and other advantages, features, and objects of the present invention will be more readily understood in view of the following detailed description and the drawings.

**BRIEF DESCRIPTION OF THE DRAWINGS**

The present invention can be more readily understood in conjunction with the accompanying drawings, in which:

FIG. 1 is a flowchart of the enrollment mode of the present invention.
FIG. 2 is a flowchart of the identity authentication mode of the present invention.
FIG. 3 is a flowchart of the "acquire trial" procedure.

FIG. 4 is a flowchart of the procedure used to enroll a new client.

FIG. 5 is a flowchart of the identity authentication mode of the present invention.

FIG. 6 is a graph showing pulse waveform exemplar shape vectors of the ten subjects of a recent study, along with the mean pulse waveform.

FIG. 7 is a graph showing an example of an enrollment trial with railed data.

## DETAILED DESCRIPTION OF THE INVENTION

The present invention provides a biometric system for characterizing individuals by the non-invasive sensing of the subject's pulse waveform for the purposes of identification and identity tracking/confirmation. The major components include a computer processor, data storage, and a pulse sensor adjacent to the subject's tissue that generates time-series data based on the subject's pulse waveforms.

As an overview, the processor initially receives and analyzes the pulse waveform data from the pulse sensor for a known subject to generate subject characterization data identifying the known subject. Thereafter, in the identity authentication mode, the processor receives data from the pulse sensor for a test subject, who may or may not be the known subject. The processor analyzes this data in conjunction with the stored subject characterization data to determine whether the test subject is the same as the known subject. For the purposes of this application, it should be understood that the phrase "test subject" refers to the person whose identity is being tested or confirmed during the identity authentication mode of the present system.

Thus, the present system operates in one of two mutually exclusive modes - an enrollment mode and an identity authentication mode. The enrollment mode acquires subject data under the supervision of a trained technician, computes subject characteristics, calculates the probability of an impostor producing similar characteristics, and stores these findings in a client database for later use during the identity authentication mode.

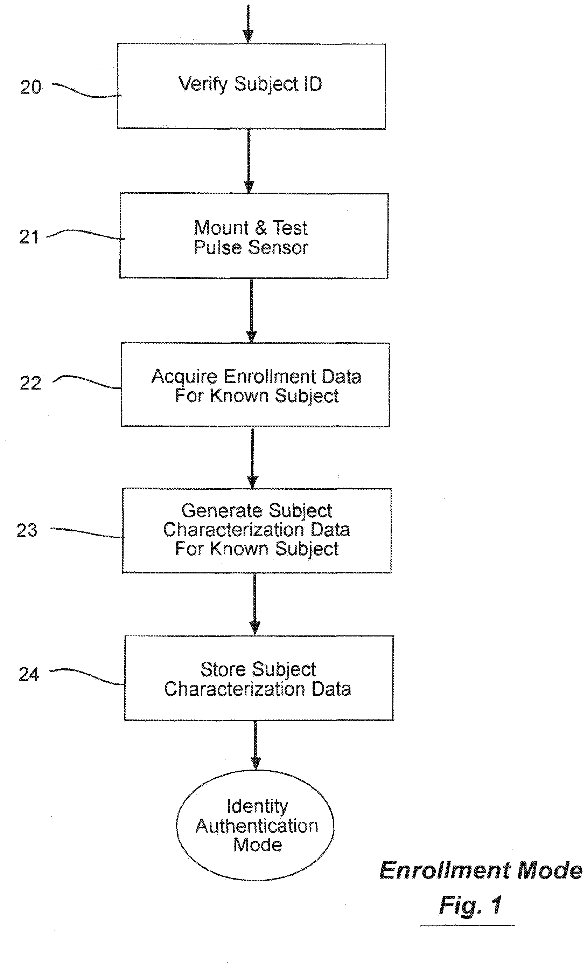

Figure 1 is a general flowchart of the enrollment mode employed to initially build subject characterization data for a known subject. The operator first verifies the pre-processes a short, contiguous time-series data of the digitized measurement. called a "trial".

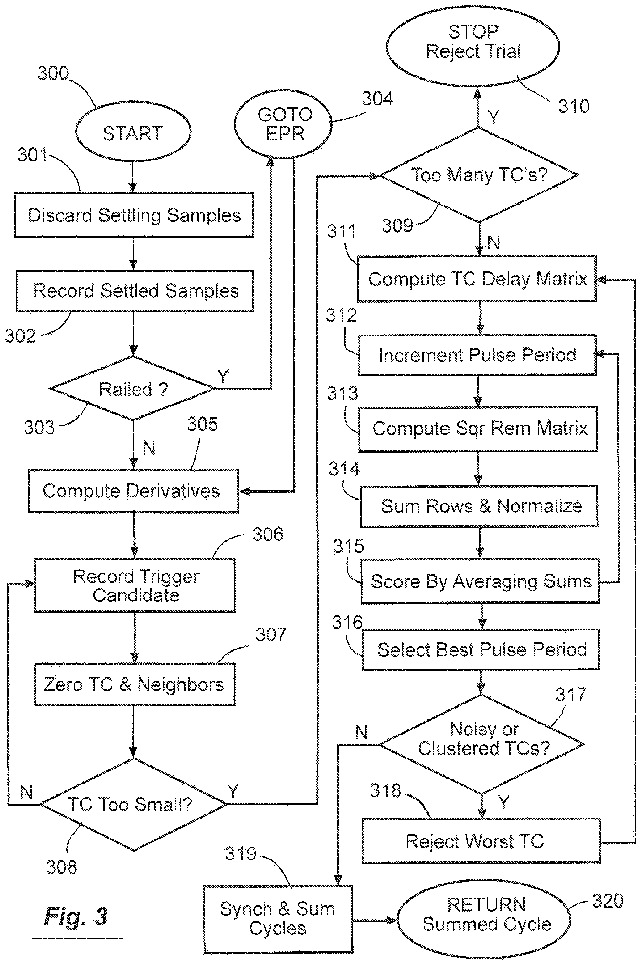

Figure 3 shows how the present system acquires a trial. The trial pulse waveform typically consists of a small number (e.g., ten) of pulse cycles, which are similar but not identical to each other. Performance is improved by synchronizing and summing pulse cycles to wash out noise. The goal of the procedure is to convert the multi-cycle waveform into a single representative cycle, or exemplar. Block 300 is the start of the procedure. Block 301 reads and discards pulse samples for a fixed duration (e.g., 1.5 seconds) while the waveform settles. After settling, block 302 reads and records samples for the remainder of the trial (e.g., 8.5 seconds).

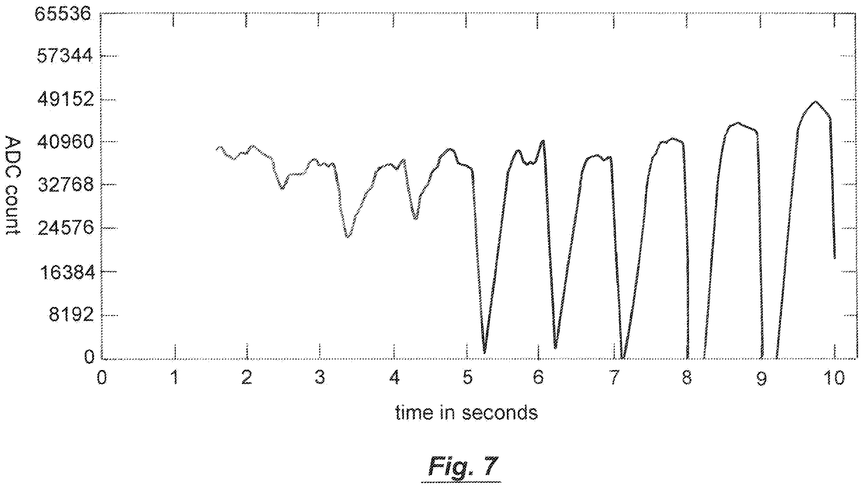

Block 303 tests samples for "railing" (i.e.. exceeding the limits of the digitizer, an indicator of trial corruption) as shown for example in Figure 7. Upon detecting a railed sample, block 303 calls block 304, the extrapolative peak restoration routine, which is discussed below. If no recorded samples are railed, or railed peaks are reparable, control proceeds to block 305, which calculates the first and second derivatives of the pulse wave with respect to time, to eliminate baseline drift and generate triggers associated with the systolic excursion. Representing the subject's pulse wave with its first derivative also obscures the bio-informational nature of the signal, thus enhancing privacy. The derivatives may or may not be smoothed to reduce high frequency noise. Block 306 chooses the most negative excursion of the second derivative as the "trigger candidate" (TC). Next, block 307 zeroes the TC and some small number (e.g., four) of immediate predecessor and successor data, to avoid selecting the same peak again. Then, block 308 compares the present TC to the first TC. if the present TC is greater than some threshold fraction (e.g., ½) of the first TC, the procedure loops through blocks 306-308, acquiring another TC. If not, TC acquisition is deemed complete, and control proceeds to block 309. If there are many (e.g., eight) more TCs than can be accounted for according to the settled sampling time and maximum pulse rate (e.g., 17 for 8.5 seconds at 120 beats per The processor analyzes the enrollment data and generates subject characterization data for identifying the known subject (step 23). This subject characterization data is stored for later use during the identity authentication mode of the present system (step 24), as will be described below.

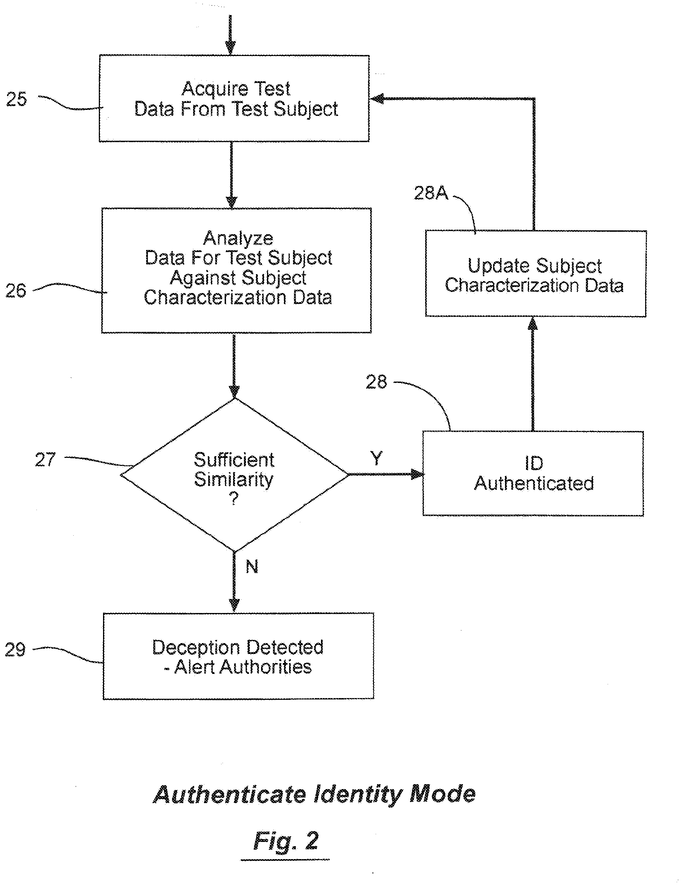

The identity authentication mode is used to authenticate the identity of a test subject, who may or may not be the known subject from the enrollment mode. In this mode, the system acquires subject data unsupervised in the field, compares it to subject and impostor characteristics, and decides whether to authenticate or challenge identification. Figure 2 is a flowchart of one possible embodiment of the identity authentication mode. For each identity authentication test, the processor acquires pulse waveform data from the pulse sensor for the test subject (step 25). The processor analyzes this test data using the subject characterization data (step 26). Based on this analysis, in step 27, the processor determines whether there is a sufficient degree of similarity between the pulse waveform characteristics of the known subject (from the subject characterization data) and the test subject to conclude that these subjects are the same person (step 28). If so, the processor may update the subject characterization data 18 to include the current test data (step 28A) and then loop back to step 25. Otherwise, if the processor determines that the current test subject is not the same as the known subject, an alarm can be activated to signal that deception has been detected (step 29).

As will be discussed below, the two modes in the preferred embodiment of the present invention share a common "acquire trial" procedure that acquires and pre-processes a short, contiguous time-series data of the digitized measurement, called a "trial".

Figure 3 shows how the present system acquires a trial. The trial pulse waveform typically consists of a small number (*e.g.,* ten) of pulse cycles, which are similar but not identical to each other. Performance is improved by synchronizing and summing pulse cycles to wash out noise. The goal of the procedure is to convert the multi-cycle waveform into a single representative cycle, or exemplar. Block 300 is the start of the procedure. Block 301 reads and discards pulse samples for a fixed duration (*e.g.*, 1.5 seconds) while the waveform settles. After settling, block 302 reads and records samples for the remainder of the trial (*e.g.*, 8.5 seconds).

Block 303 tests samples for "railing" (*i.e.*, exceeding the limits of the digitizer, an indicator of trial corruption) as shown for example in Figure 7. Upon detecting a railed sample, block 303 calls block 304, the extrapolative peak restoration routine, which is discussed below. If no recorded samples are railed, or railed peaks are reparable, control proceeds to block 305, which calculates the first and second derivatives of the pulse wave with respect to time, to eliminate baseline drift and generate triggers associated with the systolic excursion. Representing the subject's pulse wave with its first derivative also obscures the bio-informational nature of the signal, thus enhancing privacy. The derivatives may or may not be smoothed to reduce high frequency noise. Block 306 chooses the most negative excursion of the second derivative as the "trigger candidate" (TC). Next, block 307 zeroes the TC and some small number (e.g., four) of immediate predecessor and successor data, to avoid selecting the same peak again. Then, block 308 compares the present TC to the first TC. If the present TC is greater than some threshold fraction (*e.g.*, ½) of the first TC, the procedure loops through blocks 306-308, acquiring another TC. If not, TC acquisition is deemed complete, and control proceeds to block 309. If there are many (*e.g.*, eight) more TCs than can be accounted for according to the settled sampling time and maximum pulse rate (*e.g.,* 17 for 8.5 seconds at 120 beats per minute), the sample is judged too noisy, and block 309 calls block 310, which rejects the trial and stops the procedure. If not, the trial is accepted, but some of the TCs may be noise spikes asynchronous to the underlying pulse cycle.

The section labeled as blocks 311-318 is called the "trigger sieve" because it removes asynchronous false triggers, thus enhancing performance. Block 311 calculates a square matrix of the delays Δ between every pair of TCs. Next, the procedure loops through all integer pulse periods, in units of the sampling period, from the fastest to the slowest measurable pulse *(e.g.,* 50-150 for 100 Hz sampling and 120-40 beats per minute), to find the best fit to the preponderance of TCs. Block 312 increments the pulse period P. Block 313 computes the matrix of squared remainders $[\Delta \bmod P]^2$, where the "mod" operation yields the integer remainder with the smallest absolute value (*e.g.* 15 mod 8 equals -1, not 7). Block 314 sums the squared remainders for each TC relative to the other TCs, and normalizes such that a "score" near (much smaller than) unity indicates P is a poor (good) fit to the true pulse period. Block 315 averages the TC scores to evaluate P's goodness of fit. Block 316 selects the P with the lowest score. Next, block 317 detects if there are TCs with optimal-P scores greater than a preset threshold (e.g., 0.25), or any clustered TCs, as false triggers not synchronized with the prevailing pulsatile rhythm. If there is at least one false trigger, block 318 eliminates the TC with the largest optimal-P score, and control returns to block 311. If there are no false triggers, block 319 uses the true triggers to synchronize and sum the cycles, and block 320 returns the summed cycle to the calling program.

The following is a discussion of the procedure for extrapolative peak restoration (EPR) of clipped or railed pulse wave peaks in block 304. When prototyped, the prototype hardware appeared to have sufficient range to capture pulse wave data without overdriving the analog-to-digital convertor, or ADC. This is also known as clipping or railing. Subsequently, some strong-signal subjects have demonstrated pulse waves whose peaks are beyond the limits of the ADC, as shown for example in Figure 7. One possible rule is to discard any trial that rails after initial settling, potentially resulting in much spoiled data. The alternative of reducing the sensitivity so that even the strongest pulses remain within bounds is unattractive because we also have several weak-signal subjects whose pulse wave can scarcely be discerned as it is. Therefore, we desire a method for the extrapolative restoration of the clipped pulse wave peaks.

Figure 7 shows an example of the data used to develop this model. This represents about the worst trial that should be restored (any trial with worse railing should be discarded). Data from the initial settling (the first 1.5 seconds) is not shown. There are three clipped waves, with widths of 9, 22, and 19 consecutive ADC counts of zero respectively, for fifty total railed data. Clipping at the other rail 65535 is also possible, of course.

One embodiment of the present invention employs the following criteria: A railed trial should be restored if it has: (1) no clipped peak of width greater than 25; (2) tails sufficiently long for restoration on both sides of each clipped peak; and (3) has a total clipped width no greater than 85 (10% of the post-settling data). Otherwise, it should be discarded. The method uses a Gaussian-weighted parabolic best fit. It marches through the data set, separately restoring each clipped peak in chronological order. One may as well specify fit window as wide as the exemplar (e.g. 49 data), and symmetrical about the mid-point of the railed segment of length L: $W(k) = exp\left[-\dfrac{k^2}{2\sigma^2}\right]$ for $|k| > \frac{1}{2}(L-1)$ if L is odd, and for $|k| > \frac{1}{2}L$ if L is even, and $W(k) = 0$ for k otherwise; where (*e.g.* $k \in [-24,+24]$) is defined relative to the center-most datum if L is odd, and to the earlier member of the center-most pair of data if L is even; and $\sigma$ is a user-selected standard deviation (*e.g.,* eight). The best fit $\tilde{Z}(k) = Ak^2 + Bk + C$ is found by optimizing the fit coefficients using weighted least-squares. Because of symmetry, sums over odd powers of k vanish, yielding:

$$\begin{bmatrix} \Sigma Wk^4 & 0 & \Sigma Wk^2 \\ 0 & \Sigma Wk^2 & 0 \\ \Sigma Wk^2 & 0 & \Sigma W \end{bmatrix} \bullet \begin{bmatrix} A \\ B \\ C \end{bmatrix} = \begin{bmatrix} \Sigma Wk^2 Z \\ \Sigma WkZ \\ \Sigma WZ \end{bmatrix}$$

where $\Sigma$ is a sum over k. Inverting this gives:

$$B = \frac{\Sigma WkZ}{\Sigma Wk^2} \ and \ \begin{bmatrix} A \\ C \end{bmatrix} = \frac{1}{\Sigma Wk^4 \Sigma W - (\Sigma Wk^2)^2} \begin{bmatrix} \Sigma W & -\Sigma Wk^2 \\ -\Sigma Wk^2 & \Sigma Wk^4 \end{bmatrix} \bullet \begin{bmatrix} \Sigma Wk^2 Z \\ \Sigma WZ \end{bmatrix}$$

Quantities independent of Z can be evaluated beforehand, and stored in a look-up table. Sums over products containing Z still require explicit computation. Once A, B, and C have been computed, the best fit $\tilde{Z}(k) = Ak^2 + Bk + C$ can be calculated for $k \in$ [-24,+24], and the following rule can be applied to restore the clipped peak: $Z(k) \leftarrow extremum(\tilde{Z}(k), \tilde{Z}(k))$, (*i.e.,* choose the more extreme of the fit and measured values, independently for each k). This will tend to use the fit values across the clipped segment as desired, but revert to the measured values on the tails of the fit window, where the parabolic fit is poor.

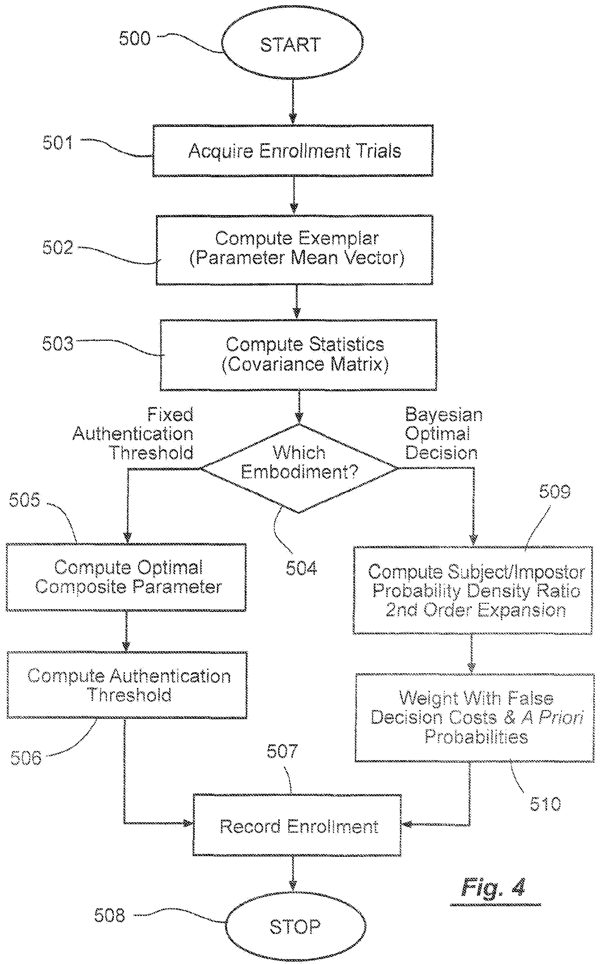

Figure 4 shows two embodiments of the procedure used to enroll a new client. This procedure can be used both to establish the client's characteristics as a subject whose identity will be putative in the field, and as a possible impostor for any other client. Block 500 is the start of the procedure. Block 501 acquires a number of trial time periods (*e.g.,* five or seven) by repeatedly calling the appropriate "Acquire Trial" procedure. Block 502 computes the exemplar (*i.e.,* the arithmetic mean over the enrollment trials of any or all of the pulse wave shape vector and possibly other parameters, arranged into a vector) using some or all of the enrollment trials.

In one embodiment of the present invention, only the pulse waveform data from the most consistent trials are averaged to create the exemplar according to a predetermined rule in block 502. For example, the pulse waveform data for the five most-typical trials out of seven total trials can be averaged to create the exemplar. In other words, the two most atypical trials are discarded. In another embodiment, the entire set of enrollment trials for a subject can be rejected as being substandard and the subject is then required to repeat the enrollment trial process. For example, the enrollment trials for a subject can be determined to be substandard if the dynamically-weighted variance of the pulse waveform data exceeds a predetermined threshold.

After block 502 has computed the exemplar, block 503 computes the statistics (i.e., the covariance matrix) of the enrollment trial, as well as the relative weights of the shape vector components. The latter may incorporate either or both of two independent innovations: dynamic weighting, in which portions of the shape vector that are more repeatable from trial to trial are accentuated relative to less repeatable portions; and feature weighting, in which portions of the shape vector that are more specific to the subject are accentuated relative to portions more typical of the population at large.

Block 504 transfers control to one of two blocks, depending on whether the "fixed authentication threshold" or the "Bayesian optimal decision" embodiment of the algorithm is selected. The chief distinction is that the Bayesian embodiment makes use of potential impostor data (*i.e.,* from other clients as potentials impostors for the subject), while the fixed threshold does not. Block 505 finds the principal components of the covariance matrix, and uses the dominant eigenvector (*i.e.,* that with the largest eigenvalue) to linearly combine the parameter vector into a scalar "composite parameter", which is optimal in the sense that the enrollment data has the greatest correlation, and thus the least spread, along the dominant eigenvector. In general, this results in unequal weighting of the parameters in the decision to authenticate or challenge identity. Next, block 506 computes the authentication threshold corresponding to the preset desired true authentication probability (e.g., ⅞). Then, block 507 enrolls the client, and block 508 stops the procedure. On the other bifurcation, block 509 expands the ratio of the subject probability density to the impostor probability density to second order in the deviation from the subject exemplar. Block 510 includes the effects of the generally unequal penalties of false authentication and false challenge, and the *a priori* probability of attempted deception, which varies among clients. Since the Bayesian optimal decision embodiment uses the entire covariance matrix, it is not necessary or advantageous to define a composite parameter; and since impostor data is incorporated, the true and false authentication probabilities can be traded.

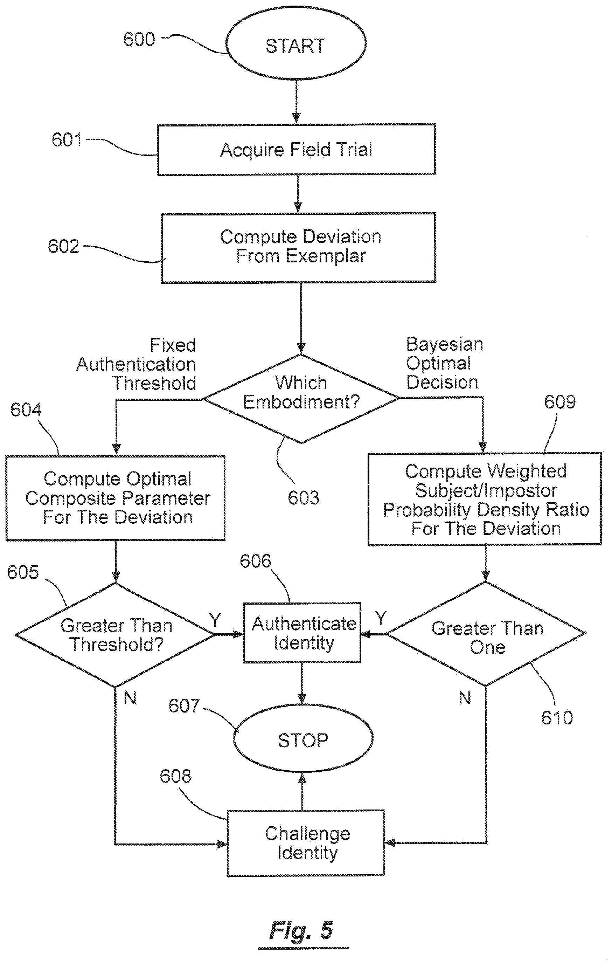

Figure 5 shows how either algorithm embodiment decides whether to authenticate or challenge the subject's identity based on a field trial. Block 600 is the start of the procedure. Block 601 acquires a field trial, and block 602 subtracts the subject exemplar to yield the "deviation", a vector with the same structure as a trial, and optionally applies dynamic or feature weighting to the deviations of the shape vectors. Block 603 transfers control to one of two blocks, depending on whether the "fixed authentication threshold" or the "Bayesian optimal decision" embodiment of the algorithm is selected. Block 604 computes the optimal composite parameter for the deviation, and block 605 compares it to the authentication threshold. If greater, block 606 advises authorities to authenticate the subject's identity, and block 607 stops the procedure. If lesser, block 608 advises authorities to challenge the subject's identity. On the other bifurcation, block 609 computes the ratio of the subject probability density to the impostor probability density to second order in the deviation of the field trial from the subject exemplar, and block 610 compares it to one. If greater than one, block 606 advises authorities to authenticate the subject's identity. If not, block 608 advises authorities to challenge the subject's identity.

As so far described, the algorithm uniformly weights each exemplar shape vector component, placing equal importance on the various features. However, this restriction is unnecessary, and may not be optimal. Some parts of some subjects' exemplars are more characteristic than other parts, so it's reasonable to suppose weighting unusual features more heavily could enhance the distinguishability of subjects.

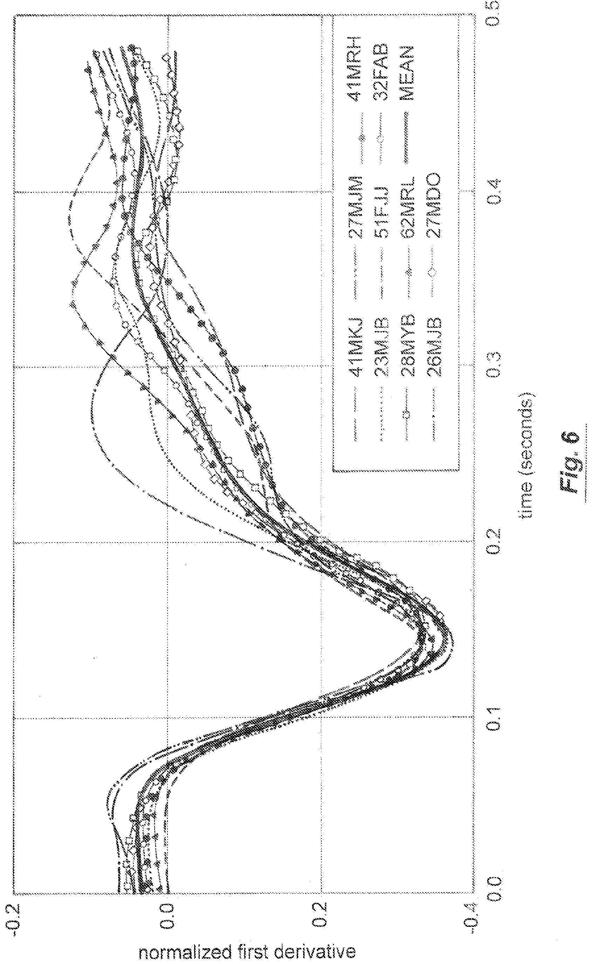

Figure 6 shows the pulse wave exemplar shape vectors of the ten subjects of a recent study, along with the mean pulse wave shape. Generally, some subjects are more atypical than others, and therefore are more easily identified in the field. Some subjects have features (e.g., subject 26MJB near 0.27 seconds) that are quite distinctive. If these features are weighted more heavily than more typical regions (e.g., subject 26MJB near 0.14 seconds), the subject is more readily recognized when supplying a legitimate field trial, and less easily mimicked by an impostor. An example feature-weighting strategy is to weight each field trial shape vector component proportionally to the square of the deviation of the corresponding subject exemplar component from the mean exemplar component, thus placing greater weight on more unusual features.

One technique for implementing dynamic weighting is to parse the shape vector into segments that are large enough to avoid excessive statistical fluctuations, yet small enough to provide resolution of the varying character across the vector (e.g., a 100-component pulse waveform vector into 20 five-component segments), and assign a different weight to each segment based on its fluctuations. An example dynamic-weighting strategy is to weight each field trial shape vector segment proportionally to the reciprocal of the segment's variance (i.e., the sum over enrollment trials and segment components of the squared deviation of the enrollment trial component from the exemplar component), thus placing greater weight on more repeatable segments.

One technique for implementing feature weighting is to raise each shape vector component probability to a different power greater or less than unity, according to how much the exemplar shape deviates from the average subject at that point. The feature weighting function can be expressed as a vector of the same dimensionality as the shape itself, consisting of components whose average is unity (equal weighting is encompassed as the special case where all components are one). This approach keeps the rest of the algorithm unaffected by whether feature weighting is selected or disabled. In general, the feature weighting vector is different for each client.

The above disclosure sets forth a number of embodiments of the present invention described in detail with respect to the accompanying drawings. Those skilled in this art will appreciate that various changes, modifications, other structural arrangements, and other embodiments could be practiced under the teachings of the present invention without departing from the scope of this invention as set forth in the following claims.

**Claims**

1. A method for biometric identity confirmation of a subject having a pulse, said method comprising:

   during an initial training mode,

      acquiring (22) pulse waveform data from a known subject;
      generating (23) and
      storing (24) subject characterization data for the known subject based at least in part on an exemplar created by
      synchronous averaging of pulse waveform data over multiple pulse cycles;

   and during a subsequent identity authentication mode,

      acquiring (25) pulse waveform data from a test subject, and
      analyzing (26) the pulse waveform data with the subject characterization data for the known subject to

confirm (27, 28) whether the identity of the test subject matches the known subject; **characterized in that:** during the initial training mode, the synchronous averaging of pulse waveform data includes:

> (a) identifying trigger candidates (306) for a start point of each pulse cycle, wherein the trigger candidates are derived at least in part from a second derivative (305) of the pulse waveform data with respect to time;
> (b) analyzing a time delay between trigger candidates to discard asynchronous false trigger candidates and identify true trigger candidates (311 - 318); and
> (c) synchronously averaging (319) the pulse waveform data for each pulse cycle using the true trigger candidates as start points for each pulse cycle.

2. The method of claim 1 wherein the step of acquiring and analyzing pulse waveform data for a test subject further comprises:

> acquiring (25) pulse waveform data from a test subject over multiple pulse cycles;
> identifying (306) trigger candidates for the start point of each pulse cycle;
> analyzing the time delay between trigger candidates to discard false trigger candidates and identify true trigger candidates (311 - 318); and
> synchronously averaging (319) the pulse waveform data for each pulse cycle using the true trigger candidates as the start points for each pulse cycle.

3. The method of claim 1 wherein the step of generating subject characterization data further comprises:

> computing an exemplar (502) in the form of a parameter vector from the pulse waveform data for the known subject;
> computing a covariance matrix (503) from the pulse wave data for the known subject;
> computing an optimal composite parameter (505) from the covariance matrix and parameter vector that is characteristic of the known subject; and
> computing an authentication threshold (506) corresponding to a desired true authentication probability for the known subject.

4. The method of claim 1 wherein the step of analyzing the pulse waveform data with the subject characterization data for the known subject to confirm whether the identity of the test subject matches the known subject further comprises:

> computing a deviation (602) of the pulse waveform data for the test subject from the exemplar for the known subject;
> computing an optimal composite parameter (604) from the deviation; and
> confirming the identity (606) of the test subject matches the known subject if optimal composite parameter is greater than the authentication threshold for the known subject (605).

5. The method of claim 1 wherein the step of generating subject characterization data further comprises:

> computing an exemplar (502) in the form of a parameter vector from the pulse waveform data for the known subject;
> computing a covariance matrix (503) from the pulse waveform data for the known subject; and
> computing a probability distribution ratio (509) of a weighted subject/impostor probability density by a Bayesian optimal decision analysis of the parameter vector, covariance matrix, and data from other subjects as potential impostors for the known subject.

6. The method of claim 1 wherein the step of analyzing the pulse waveform data with the subject characterization data for the known subject to confirm whether the identity of the test subject matches the known subject further comprises:

> computing a deviation (602) of the pulse waveform data for the test subject from the exemplar for the known subject;
> computing a weighted subject/impostor probability density ratio (609) for the deviation; and
> confirming the identity (606) of the test subject matches the known subject if the weighted subject/impostor probability density ratio is greater than one (610).

7. The method of claim 1 wherein the step of generating subject characterization data further comprises weighting portions of the exemplar selected based on their repeatability observed during the initial training mode.

8. The method of claim 1 wherein the step of generating subject characterization data further comprises weighting portions of the exemplar selected to distinguish characteristic features of the known subject observed during the initial training mode.

9. The method of claim 1 wherein the initial training mode acquires a plurality of sets of pulse waveform data over a plurality of trials, and wherein the step of synchronous averaging includes only the pulse waveform data from the most consistent trials.

10. The method of claim 1 wherein the initial training mode acquires a plurality of sets of pulse waveform data over a plurality of trials, and wherein the trials are rejected if the variance of the pulse waveform data exceeds a predetermined threshold.

**Patentansprüche**

1. Verfahren zur biometrischen Identitätsbestätigung eines Subjekts, das einen Puls aufweist, wobei das Verfahren umfasst:

während eines anfänglichen Trainingsmodus:

Erfassen (22) von Pulswellenformdaten von einem bekannten Subjekt;
Generieren (23) und
Speichern (24) von Subjektcharakterisierungsdaten für das bekannte Subjekt mindestens teilweise basierend auf einem Muster, das durch synchrone Mittelwertbildung von Pulswellenformdaten über mehrere Pulszyklen erzeugt wurde;

und während eines nachfolgenden Identitätsauthentifizierungsmodus:

Erfassen (25) von Pulswellenformdaten von einem Testsubjekt, und
Analysieren (26) der Pulswellenformdaten mit den Subjektcharakterisierungsdaten für das bekannte Subjekt zum
Bestätigen (27, 28), ob die Identität des Testsubjekts dem bekannten Subjekt entspricht;

**dadurch gekennzeichnet, dass:**
während des anfänglichen Trainingsmodus die synchrone Mittelwertbildung der Pulswellenformdaten einschließt:

(a) Identifizieren von Triggerkandidaten (306) für einen Startpunkt jedes Pulszyklus, wobei die Triggerkandidaten mindestens teilweise von einer zweiten Ableitung (305) der Pulswellenformdaten nach der Zeit abgeleitet sind;
(b) Analysieren einer zeitlichen Verzögerung zwischen Triggerkandidaten, um asynchrone falsche Triggerkandidaten zu verwerfen und wahre Triggerkandidaten (311 bis 318) zu identifizieren; und
(c) synchrone Mittelwertbildung (319) der Pulswellenformdaten für jeden Pulszyklus unter Verwendung der wahren Triggerkandidaten als Startpunkte für jeden Pulszyklus.

2. Verfahren nach Anspruch 1, wobei der Schritt des Erfassens und Analysierens von Pulswellenformdaten für ein Testsubjekt des Weiteren umfasst:

Erfassen (25) von Pulswellenformdaten von einem Testsubjekt über mehrere Pulszyklen;
Identifizieren (306) von Triggerkandidaten für den Startpunkt jedes Pulszyklus;
Analysieren der zeitlichen Verzögerung zwischen Triggerkandidaten, um falsche Triggerkandidaten zu verwerfen und wahre Triggerkandidaten (311 bis 318) zu identifizieren; und
synchrone Mittelwertbildung (319) der Pulswellenformdaten für jeden Pulszyklus unter Verwendung der wahren Triggerkandidaten als die Startpunkte für jeden Pulszyklus.

3. Verfahren nach Anspruch 1, wobei der Schritt des Generierens von Subjektcharakterisierungsdaten des Weiteren umfasst:

Berechnen eines Musters (502) in Form eines Parametervektors aus den Pulswellenformdaten für das bekannte Subjekt;

Berechnen einer Kovarianzmatrix (503) aus den Pulswellendaten für das bekannte Subjekt;

Berechnen eines optimalen zusammengesetzten Parameters (505) aus der Kovarianzmatrix und einem Parametervektor, der für das bekannte Subjekt charakteristisch ist; und

Berechnen eines Authentifizierungsschwellenwerts (506), welcher einer gewünschten wahren Authentifizierungswahrscheinlichkeit für das bekannte Subjekt entspricht.

4. Verfahren nach Anspruch 1, wobei der Schritt des Analysierens der Pulswellenformdaten mit den Subjektcharakterisierungsdaten für das bekannte Subjekt, um zu bestätigen, ob die Identität des Testsubjekts dem bekannten Subjekt entspricht, des Weiteren umfasst:

Berechnen einer Abweichung (602) der Pulswellenformdaten für das Testsubjekt aus dem Muster für das bekannte Subjekt;

Berechnen eines optimalen zusammengesetzten Parameters (604) aus der Abweichung; und

Bestätigen, dass die Identität (606) des Testsubjekts derjenigen des bekannten Subjekts entspricht, falls der optimale zusammengesetzte Parameter größer als der Authentifizierungsschwellenwert für das bekannte Subjekt (605) ist.

5. Verfahren nach Anspruch 1, wobei der Schritt des Generierens von Subjektcharakterisierungsdaten des Weiteren umfasst:

Berechnen eines Musters (502) in Form eines Parametervektors aus den Pulswellenformdaten für das bekannte Subjekt;

Berechnen einer Kovarianzmatrix (503) aus den Pulswellenformdaten für das bekannte Subjekt; und

Berechnen eines Wahrscheinlichkeitsverteilungsverhältnisses (509) einer gewichteten Subjekt/Täuscher-Wahrscheinlichkeitsdichte durch eine bayessche optimale Entscheidungsanalyse des Parametervektors, der Kovarianzmatrix und Daten von anderen Subjekten als potentielle Täuscher für das bekannte Subjekt.

6. Verfahren nach Anspruch 1, wobei der Schritt des Analysierens der Pulswellenformdaten mit den Subjektcharakterisierungsdaten für das bekannte Subjekt, um zu bestätigen, ob die Identität des Testsubjekts dem bekannten Subjekt entspricht, des Weiteren umfasst:

Berechnen einer Abweichung (602) der Pulswellenformdaten für das Testsubjekt aus dem Muster für das bekannte Subjekt;

Berechnen eines gewichteten Subjekt/Täuscher-Wahrscheinlichkeitsdichteverhältnisses (609) für die Abweichung; und

Bestätigen, dass die Identität (606) des Testsubjekts dem bekannten Subjekt entspricht, falls das gewichtete Subjekt/Täuscher-Wahrscheinlichkeitsdichteverhältnis größer als eins (610) ist.

7. Verfahren nach Anspruch 1, wobei der Schritt des Generierens von Subjektcharakterisierungsdaten des Weiteren Gewichten von Anteilen des Musters umfasst, die basierend auf ihrer Wiederholbarkeit ausgewählt wurden, die während des anfänglichen Trainingsmodus beobachtet wurde.

8. Verfahren nach Anspruch 1, wobei der Schritt des Generierens von Subjektcharakterisierungsdaten des Weiteren Gewichten von Anteilen des Musters umfasst, die ausgewählt wurden, um charakteristische Merkmale des bekannten Subjekts zu unterscheiden, die während des anfänglichen Trainingsmodus beobachtet wurden.

9. Verfahren nach Anspruch 1, wobei der anfängliche Trainingsmodus eine Vielzahl von Sätzen von Pulswellenformdaten über eine Vielzahl von Versuchen umfasst, und wobei der Schritt der synchronen Mittelwertbildung nur die Pulswellenformdaten aus den konsistentesten Versuchen einschließt.

10. Verfahren nach Anspruch 1, wobei der anfängliche Trainingsmodus eine Vielzahl von Sätzen von Pulswellenformdaten über eine Vielzahl von Testläufen umfasst, und wobei die Testläufe abgelehnt werden, falls die Varianz der Pulswellenformdaten einen vorbestimmten Schwellenwert überschreitet.

**Revendications**

1. Procédé de confirmation d'identité biométrique d'un sujet ayant une impulsion, ledit procédé comprenant :

pendant un mode d'apprentissage initial, l'acquisition (22) de données de forme d'onde d'impulsion d'un sujet connu ;
la génération (23) et
le stockage (24) de données de caractérisation de sujet pour le sujet connu, basées au moins en partie sur un exemplaire créé en faisant la moyenne synchrone de données de forme d'onde d'impulsion sur de multiples cycles d'impulsion ; et
pendant un mode d'authentification d'identité ultérieur,
l'acquisition (25) de données de forme d'onde d'impulsion à partir d'un sujet de test, et
l'analyse (26) des données de forme d'onde d'impulsion avec les données de caractérisation de sujet pour le sujet connu pour
confirmer (27, 28) si l'identité du sujet de test correspond au sujet connu ; **caractérisé en ce que** : pendant le mode d'apprentissage initial, la moyenne synchrone de données de forme d'onde d'impulsion comprend : (a) l'identification de candidats de déclenchement (306) pour un point de départ de chaque cycle d'impulsion, les candidats de déclenchement étant dérivés au moins en partie d'une dérivée seconde (305) des données de forme d'onde d'impulsion par rapport au temps ; (b) l'analyse d'un délai entre les candidats de déclenchement pour rejeter les faux candidats de déclenchement asynchrones et identifier les vrais candidats de déclenchement (311 - 318) ; et (c) la moyenne synchrone (319) des données de forme d'onde d'impulsion pour chaque cycle d'impulsion en utilisant les vrais candidats de déclenchement comme points de départ pour chaque cycle d'impulsion.

2. Procédé selon la revendication 1, l'étape d'acquisition et d'analyse de données de forme d'onde d'impulsion pour un sujet de test comprenant en outre :

l'acquisition (25) de données de forme d'onde d'impulsion d'un sujet de test sur de multiples cycles d'impulsion ;
l'identification (306) de candidats de déclenchement pour le point de départ de chaque cycle d'impulsion ;
l'analyse du délai entre les candidats de déclenchement pour rejeter des faux candidats de déclenchement et identifier des vrais candidats de déclenchement (311 - 318) ; et
la moyenne synchrone (319) des données de forme d'onde d'impulsion pour chaque cycle d'impulsion en utilisant les vrais candidats de déclenchement comme points de départ pour chaque cycle d'impulsion.

3. Procédé selon la revendication 1, l'étape de génération de données de caractérisation de sujet comprenant en outre :

le calcul d'un exemplaire (502) sous la forme d'un vecteur de paramètre à partir des données de forme d'onde d'impulsion pour le sujet connu ;
le calcul d'une matrice de covariance (503) à partir des données d'onde d'impulsion pour le sujet connu ;
le calcul d'un paramètre composite optimal (505) à partir de la matrice de covariance et du vecteur de paramètre qui est caractéristique du sujet connu ; et
le calcul d'un seuil d'authentification (506) correspondant à une probabilité d'authentification vraie souhaitée pour le sujet connu.

4. Procédé selon la revendication 1, l'étape d'analyse des données de forme d'onde d'impulsion avec les données de caractérisation de sujet pour le sujet connu pour confirmer si l'identité du sujet de test correspond au sujet connu comprenant en outre :

le calcul d'un écart (602) des données de forme d'onde d'impulsion pour le sujet de test par rapport à l'exemplaire pour le sujet connu ;
le calcul d'un paramètre composite optimal (604) à partir de l'écart ; et
la confirmation que l'identité (606) du sujet de test correspond au sujet connu si le paramètre composite optimal est supérieur au seuil d'authentification pour le sujet connu (605).

5. Procédé selon la revendication 1, l'étape de génération de données de caractérisation de sujet comprenant en outre :

le calcul d'un exemplaire (502) sous la forme d'un vecteur de paramètre à partir des données de forme d'onde d'impulsion pour le sujet connu ;

le calcul d'une matrice de covariance (503) à partir des données de forme d'onde d'impulsion pour le sujet connu ; et

le calcul d'un rapport de distribution de probabilité (509) d'une densité de probabilité sujet/imposteur pondérée par une analyse de décision optimale bayésienne du vecteur de paramètre, d'une matrice de covariance et de données d'autres sujets en tant qu'imposteurs potentiels pour le sujet connu.

6. Procédé selon la revendication 1, l'étape d'analyse des données de forme d'onde d'impulsion avec les données de caractérisation de sujet pour le sujet connu pour confirmer si l'identité du sujet de test correspond au sujet connu comprenant en outre :

le calcul d'un écart (602) des données de forme d'onde d'impulsion pour le sujet de test par rapport à l'exemplaire pour le sujet connu ;

le calcul d'un rapport de densité de probabilité sujet/imposteur pondérée (609) pour l'écart ; et

la confirmation que l'identité (606) du sujet de test correspond au sujet connu si le rapport de densité de probabilité sujet/imposteur pondérée est supérieur à un (610) .

7. Procédé selon la revendication 1, l'étape de génération de données de caractérisation de sujet comprenant en outre la pondération de parties de l'exemplaire sélectionné sur la base de leur répétabilité observée pendant le mode d'apprentissage initial.

8. Procédé selon la revendication 1, l'étape de génération de données de caractérisation de sujet comprenant en outre la pondération de parties de l'exemplaire sélectionnées pour distinguer des traits caractéristiques du sujet connu observés pendant le mode d'apprentissage initial.

9. Procédé selon la revendication 1, le mode d'apprentissage initial faisant l'acquisition d'une pluralité d'ensembles de données de forme d'onde d'impulsion sur une pluralité d'essais, et l'étape de moyenne synchrone comprenant uniquement les données de forme d'onde d'impulsion provenant des essais les plus cohérents.

10. Procédé selon la revendication 1, le mode d'apprentissage initial faisant l'acquisition d'une pluralité d'ensembles de données de forme d'onde d'impulsion sur une pluralité d'essais, et les essais étant rejetés si la variance des données de forme d'onde d'impulsion dépasse un seuil prédéterminé.

20 — Verify Subject ID

21 — Mount & Test Pulse Sensor

22 — Acquire Enrollment Data For Known Subject

23 — Generate Subject Characterization Data For Known Subject

24 — Store Subject Characterization Data

Identity Authentication Mode

*Enrollment Mode*

*Fig. 1*

**Authenticate Identity Mode**

*Fig. 2*

*Fig. 3*

500 — START

501 — Acquire Enrollment Trials

502 — Compute Exemplar
(Parameter Mean Vector)

503 — Compute Statistics
(Covariance Matrix)

Fixed
Authentication
Threshold

Bayesian
Optimal
Decision

Which
Embodiment?

504

509

505 — Compute Optimal
Composite Parameter

Compute Subject/Impostor
Probability Density Ratio
2nd Order Expansion

Compute Authentication
Threshold

Weight With False
Decision Costs & *A Priori*
Probabilities

506

510

507 — Record Enrollment

508 — STOP

*Fig. 4*

**START** — 600

601 — Acquire Field Trial

602 — Compute Deviation From Exemplar

Fixed Authentication Threshold

Bayesian Optimal Decision

Which Embodiment? — 603

604 — Compute Optimal Composite Parameter For The Deviation

609 — Compute Weighted Subject/Impostor Probability Density Ratio For The Deviation

605 — Greater Than Threshold?

606 — Authenticate Identity

610 — Greater Than One

607 — STOP

608 — Challenge Identity

_**Fig. 5**_

*Fig. 6*

*Fig. 7*

EP 3 022 613 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003135097 A

- WO 2012138663 A2